(19) Europäisches Patentamt
European Patent Office
Office européen des brevets

(11) EP 2 127 636 A1

(12) **EUROPEAN PATENT APPLICATION**
published in accordance with Art. 153(4) EPC

(43) Date of publication:
**02.12.2009 Bulletin 2009/49**

(21) Application number: **07860342.0**

(22) Date of filing: **27.12.2007**

(51) Int Cl.:
***A61K 8/60*** (2006.01) ***A61K 31/7028*** (2006.01)
***A61Q 19/00*** (2006.01) ***A61Q 19/08*** (2006.01)

(86) International application number:
**PCT/JP2007/075120**

(87) International publication number:
**WO 2008/090716 (31.07.2008 Gazette 2008/31)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.01.2007 JP 2007016535**

(71) Applicant: **Shiseido Company, Ltd.**
**Chuo-ku**
**Tokyo 104-8010 (JP)**

(72) Inventors:
- **MATSUNAGA, Yukiko**
  **Yokohama-shi**
  **Kanagawa 236-8643 (JP)**
- **AMANO, Satoshi**
  **Yokohama-shi**
  **Kanagawa 236-8643 (JP)**
- **HIRUMA, Takuya**
  **Yokohama-shi**
  **Kanagawa 224-8558 (JP)**
- **FUKUNISHI, Hirotada**
  **Yokohama-shi**
  **Kanagawa 224-8558 (JP)**
- **SUETSUGU, Masaru**
  **Yokohama-shi**
  **Kanagawa 224-8558 (JP)**
- **SHIBATA, Michio**
  **Yokohama-shi**
  **Kanagawa 236-8643 (JP)**

(74) Representative: **Adam, Holger et al**
**Kraus & Weisert**
**Patent- und Rechtsanwälte**
**Thomas-Wimmer-Ring 15**
**80539 München (DE)**

(54) **ANTI-WRINKLE AGENT AND ADAM INHIBITOR**

(57) Disclosed is an anti-wrinkle agent for ameliorating a wrinkle (particularly a fine wrinkle) developed when the barrier function is decreased by dryness and the like. Also disclosed is an ADAM inhibitor. The anti-wrinkle agent or the ADAM inhibitor comprises an alkyl glucoside represented by the general formula: (1) [wherein R represents a linear or branched alkyl group having 1 to 18 carbon atoms] or a salt thereof.

FIG.2

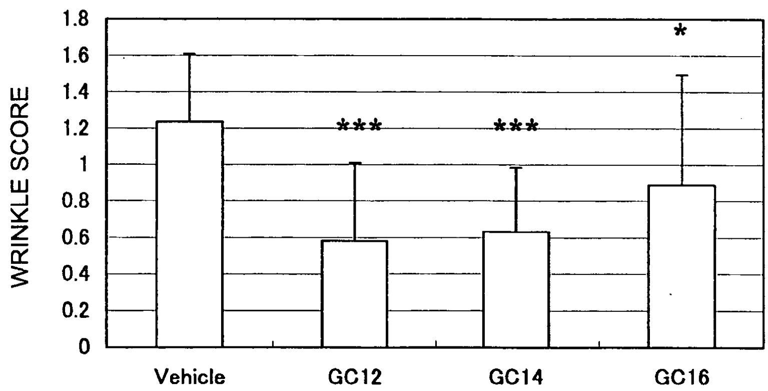


EP 2 127 636 A1

## Description

FIELD OF THE INVENTION

The present invention relates to an anti-wrinkle agent, particularly the one which prevents/ameliorates a wrinkle (particularly a fine wrinkle) resulted from the decreased barrier function by means of controlling thickening of the skin caused when the barrier function is decreased by dryness and the like. The invention also relates to a new ADAM inhibitor.

BACKGROUND ART

Recently, middle-aged and older women's interest in beauty treatment has increased, leading to increase of their concern for fine wrinkles developed at the outer corner of the eye and around the lips which become manifest due to the reduction of moisture-keeping capacity of the epidermal corneal layer (stratum corneum of the epidermis) and the reduction of the sebum with decreased lipid secretion accompanying the aging. It is considered that fine wrinkles are caused by the dehydration of the skin, and are considerably different, in the underlying mechanism and morphological, histological and biochemical changes and the like, from the large wrinkles developed from the photoaging. For example, correlation between the water content of the corneal layer and the intensity of the fine wrinkle was verified in humans (non-patent reference 1). In addition, continuous destruction of the barrier function with an unsaturated aliphatic acid reportedly developed the epidermis wrinkles (non-patent reference 2). So far in order to prevent and ameliorate such fine wrinkles, there has been no choice but to depend on protecting the skin from dehydration by means of moisturizer including glycerine, sorbitol, liquid plant extract etc. and cosmetic agents containing collagen and the like. However, protecting the skin from dehydration by means of moisturizer and the like does not suffice to prevent/ameliorate the fine wrinkles adequately, therefore more effective= prevention/amelioration has been strongly desired.

It is known that a group of membrane metalloproteases called ADAM (a disintegrin and metalloprotease) as well as matrix metalloprotease (MMP) cut various growth factors including heparin-binding epidermal growth factor-like growth factor (HB-EGF) and tumor necrosis factor (TNF), cytokines and many other membrane proteins from the cell surface and release them out of the cell resulting in proliferation and differentiation of cells by those growth factors and the like. For example, it was reported that HB-EGF expressed on the cell surface is cut by the ADAM-9, 10, 12 and 17 and released out of the cell (for example, see non-patent references 3-7).

Further, in the skin cells, expression/release from the cell membrane of growth factors including the HB-EGF, TNF-$\alpha$ and cytokines are promoted by different stimuli including the ultraviolet light, oxidization and osmotic pressure. Signals for proliferation, differentiation and the like are transmitted to the cells by the autocrine and paracrine mechanisms, leading to the proliferation and thickning of the skin. It has been known that the activation of the ADAM in the skin cell occurs during the time.

Non-Patent Reference 1: Imokawa et al., Fragrance Journal; 1992(11) 29-42.

Non-Patent Reference 2: Hitoshi Masaki, Koryokaisi; 2001, Vol. 25, No. 1. 34-38.

Non-Patent Reference 3: Asakura, M. et al., 2002, Cardiac hypertrophy is inhibited by antagonism of ADAM12 processing of HB-EGF: metalloproteinase inhibitors as a new therapy, Nat. Med. 8: 35-40.

Non-Patent Reference 4: Izumi, Y. et al., 1998, A metalloprotease-disintegrin, MDC9/meltrin-/ADAM9 and PKC are involved in TPA-induced ectodomain shedding of membrane-anchored heparin-binding EGF-like growth factor. EMBO J. 17: 7260-7272.

Non-Patent Reference 5: Lemjabbar, H. and C. Basbaum, 2002, Platelet-activating factor receptor and ADAM10 mediate responses to Staphylococcus aureus in epithelial cells. N at. Med. 8: 41-46.

Non-Patent Reference 6: Sunnarborg, S. W. et al., 2002, Tumor necrosis factor-alpha converting enzyme (TACE) regulates epidermal growth factor receptor ligand availability. J. Biol. Chem. 277: 12838-12845.

Non-Patent Reference 7: Yan, Y., K. Shirakabe, and Z. Werb, 2002, The metalloprotease Kuzbanian (ADAM10) mediates the transactivation of EGF receptor by G protein-coupled receptors, J. Cell Biol. 158: 221-226.


DISCLOSURE OF THE INVENTION

PROBLEMS TO BE SOLVED BY THE INVENTION

The present invention has been conceived in view of the above-mentioned circumstances and provides an anti-wrinkle agent and an ADAM inhibitor which have the ADAM inhibiting action and can prevent or ameliorate wrinkles (particularly fine wrinkles) effectively.

MEANS FOR SOLVING THE PROBLEM

The present inventors found that the change of the epidermis and dermis similar to the fine wrinkles in the human skin can be reproduced in the skin of the hairless mouse through the continuous destruction of the barrier by repetitive tape stripping of the mouse, and succeeded in production of a fine wrinkle mouse model. They further found that gene expression of proteins belonging to the ADAM (a disintegrin and metalloprotease) family including ADAM-9 and ADAM-17 (hereinafter, ADAM) as well as HB-EGF and Amphiregulin which are released from the cell membrane and activated by those ADAMs on the epidermis was enhanced in such a fine wrinkle mouse model, and also found that inhibition of the ADAM in the skin could surpress the thickening of the epidermis and dermis leading to prevention of wrinkle formation.

Thus, the ADAMs including ADAM-9, ADAM-10 and ADAM-17 are the enzymes which exist on the surface of the skin cell and release the growth factors including HB-EGF, Amphiregulin, TNF-$\alpha$, TGF-$\alpha$ and the like out of the cell membrane and activate them. It is considered to be an important mechanism for formation of fine wrinkles that those ADAMs are activated or their expression is enhanced in the skin by destruction of the skin barrier, which promotes release/activation of the growth factors including HB-EGF and the like leading to thickening of the epidermis and dermis. Therefore, it is presumed that suppressing the activity of the ADAM in the skin be able to control the enhancement of the activity of the HB-EGF and the like leading to control of thickening of the epidermis and dermis resulting in prevention or amelioration of wrinkles, particularly fine wrinkles. A schematic drawing shown in Fig. 1 illustrates the mechanism in which inhibition of the activity of the ADAM in the skin prevents and ameliorates wrinkles.

Based on the above findings, the present applicant has found a method wherein the skin, skin tissue or cell of the human or animal is contacted with the tested substance, then the enzymatic activity of the ADAM or the gene expression level in the skin, the tissue, or the cell is detected, and the anti-wrinkle effect of the tested substance is assessed using generation of the ADAM enzyme or the gene expression as an index. (See Japanese patent application numbers 2005-299524 and 2005-296219)

On the other hand, TAPI-1{N-(R)-(2-(Hydroxyaminocarbonyl)methyl)-4-Methylpentanoyl-L-Nal-L-Alanine2-Aminoethyl amide(L-Nal:L-3-(2'-Naphthyl)alanine)} and TAPI-2{N-(R)-(2-(Hydroxyaminocarbonyl)methyl)-4-Methylpentanoyl-L-t-Butyl-Glycyl-L-Alanine2-Aminoethyl amide} are known as the ADAM inhibitors, but the inhibitory ability of those substances could not be sufficient and their safety as the dermatological external agents has not been confirmed. Therefore, development of safer and more effective agents has been needed.

In searching for a new compound, which may inhibit the ADAM, the inventors found that certain alkyl glucosides and salts thereof have high ADAM inhibitory activity and further can inhibit formation of wrinkles markedly, thus leading to attainment of the invention.

The invention is an anti-wrinkle agent characterized by comprising an alkyl glucoside represented by the general formula described below or a salt thereof:

[Formula 1]

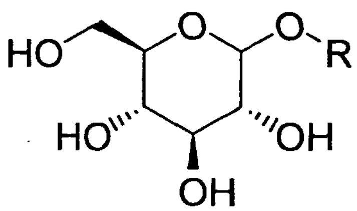

(1)

wherein, R represents a linear or branched alkyl group having 1 to 18 carbon atoms.

Further, the invention is an ADAM inhibitor characterized by comprising an alkyl glucoside represented by the general formula (1) described below or a salt thereof:

[Formula 2]

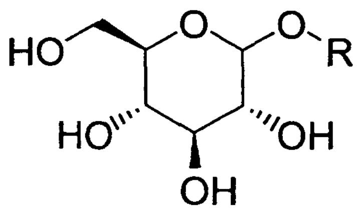

(1)

wherein, R represents a linear or branched alkyl group having 1 to 18 carbon atoms.
Furthermore, the invention is a method for preventing/ameliorating wrinkles characterized by preventing or ameliorating wrinkles by applying the ADAM inhibitor described above to the skin.

EFFECT OF THE INVENTION

The anti-wrinkle agent and ADAM inhibitor of the invention can inhibit the activity of the ADAM including ADAM-9, ADAM-10 and ADAM-17 and the like which exists in the skin and control the enhancement of the release/activation of the growth factors including HB-EGF and the like in the epidermis and dermis developed by destruction of the skin barrier caused by various factors including decrease of the sebum and face washing, leading to control of thickening of the epidermis and dermis resulting in very effective prevention from or amelioration of formation of the wrinkles, particularly the fine wrinkles.
According to the method of the invention for preventing or ameliorating the wrinkles, formation of the wrinkles (particularly the fine wrinkles) can be prevented or ameliorated by use of a new compound the anti-wrinkle effect of which has not been known.

BRIEF DESCRIPTION OF THE DRAWINGS

FIG. 1 is a schematic drawing describing the mechanism, which prevents or ameliorates wrinkles by inhibiting the ADAM activity in the epidermis;
FIG. 2 is a graph showing a wrinkle-ameliorating effect of alkyl glucosides by the visual judgement;
FIG. 3 is a graph showing an effect of alkyl glucosides for controlling increase of transepidermal Water Loss (TEWL);
FIG. 4 is a graph showing an effect of octadecyl β-D-glucoside (GC18) for ameliorating the wrinkles by the visual determination;
FIG. 5 is a graph showing an effect of octadecyl β-D-glucoside (GC18) for controlling increase of Transepidermal Water Loss (TEWL); and
FIG. 6 is a graph showing a change of the water content of the corneal layer over time for dodecyl β-D-glucoside (GC12).

BEST MODE FOR CARRYING OUT THE INVENTION

Detailed description of the invention will be described below. The alkyl glucosides or salts thereof used in the invention are represented by the general formula (1) described below.
[Formula 3]

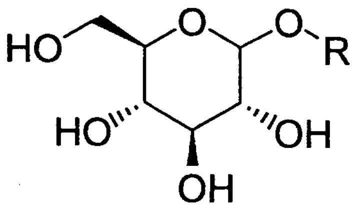

( 1 )

wherein, R represents a linear or branched alkyl group having 1 to 18 carbon atoms.
R in the alkyl glucoside (1) of the inventive compounds is a straight chain or branched $C_{1-18}$ alkyl group, preferably a linear or branched alkyl group having 10 to 18 carbon atoms, more preferably a linear or branched alkyl group having 12 to 14 carbon atoms. Such groups include, for example, methyl, ethyl, propyl, isopropyl, butyl, isobutyl, tert-butyl, pentyl, hexyl, heptyl, octyl, 2-ethylhexyl, nonyl, decyl, undecyl, dodecyl, tridecyl, tetradecyl, pentadecyl, hexadecyl, heptadecyl and octadecyl, and the like, preferably, decyl, undecyl, dodecyl, tridecyl, tetradecyl, pentadecyl, hexadecyl, heptadecyl and octadecyl, more preferably, dodecyl, tridecyl and tetradecyl.

Additionally, the inventive alkyl glucosides include, for example, methyl glucoside, ethyl glucoside, propyl glucoside, isopropyl glucoside, butyl glucoside, isobutyl glucoside, tert-butyl glucoside, pentyl glucoside, hexyl glucoside, heptyl glucoside, octyl glucoside, 2-ethylhexyl glucoside, nonyl glucoside, decyl glucoside, undecyl glucoside, dodecyl glucoside, tridecyl glucoside, tetradecyl glucoside, pentadecyl glucoside, hexadecyl glucoside, heptadecyl glucoside and octadecyl glucoside and the like.

The inventive compounds may be either $\alpha$-glucoside or $\beta$-gluoside isomer, or mixtures of $\alpha$ and $\beta$. Additionally, the glucose may be either D-glucose or L-glucose isomer or mixtures of D and L.

The alkyl glucosides of the invention include besides free acids, medically accepted salt forms thereof. These salts include, for example, alkali metal salts such as sodium and potassium salts and the like, alkaline earth metal salts such as calcium and magnesium salts and the like, amine salts such as methylamine salts, dimethylamine salts, trimethylamine salts, methylpiperidine salts, ethanolamine salts, diethanolamine salts, triethanolamine salts and lysine salts and the like, ammonium salts or basic amino acid salts, but not limited to them.

The alkyl glucosides and salts thereof can be produced according to knowm producing methods.

In addition, though the anti-wrinkle agent substantially consists of an alkyl glucoside or a salt thereof, it may contain other ingredients. This anti-wrinkle agent is mixed in the base of a dermatological agent providing a dermatological agent.

The mixing amount of the alkyl glucoside or salt thereof in the above dermatological agent is, for example, preferably 0.001-10% by mass, more preferably 0.005-5% by mass, even more preferably, 0.01-1% by mass, though not particularly limited to and varies depending on the use aspect, product form and the like.

In the specification here, the "dermatological external agent" includes cosmetics, drugs and quasi drugs, etc. Its formulations include any such as an aqueous, solubilizing, emulsified, oil, gel, past, ointment, aerosol, water-oil bilayer and water-oil-powder three layer systems. It also includes those carried on the sheeted base.

The dermatological external agent can also adopt any product forms and uses. For example, it can be used as an external agent for face, body or scalp including lotions, emulsions, creams and packs.

The dermatological external agent may mix properly as needed besides the above described alkyl glucosides or their salts, any other components used in the dermatological external agent including ordinary cosmetics and drugs, and may be produced according to the conventional methods depending on the intended formulation. For example, an alkyl glucoside or a salt thereof may be mixed with one or two or more of the ingredients below to prepare a dermatological external agent.

Ultraviolet light absorbing agents include, for example, ultraviolet absorber of benzoic acid system such as para-aminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA methyl ester and the like; ultraviolet absorber of anthranilic acid system such as homomenthyl- N-acetyl anthranilate and the like; ultraviolet absorber of salicylic acid system such as amyl salicylate, menthyl salicylate, homomenthyl salicylate, octyl salicylate, phenyl salicylate, benzyl salicylate, p-isopropanol phenyl salicylate and the like; ultraviolet absorber of cinnamic acid system such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-dipara-methoxy cinnamate, methyl bis(trimethylsiloxane)silylisopentyl trimethoxy cinnamate and the like; 3-(4'-methylbenzylidene)-d,1-camphor; 3-benzylidene-d,1-camphor; urocanic acid, urocanic acid ethyl ester; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzaladine; dianisoylmethane, 4-methoxy-4'-t-butyldibenzoylmethane; 5-(3,3-dimethyl-2-norbornylidene)-3-pentane-2-one; dimorpholinopyridazinone etc. and any one or two or more may be used.

Ultraviolet light scattering agents include, for example, powders such as titanium oxide, particulate titanium oxide, zinc oxide, particulate zinc oxide, ferric oxide, particulate ferric oxide, ceric oxide and the like.

For these ultraviolet light scattering agents, a spicular, spindle, spherical or granular powder is usually used. In addition, particulate powders which have the particle size of not more than 0.1 μm are preferable.

Also preferable are ultraviolet light scattering agents silicone-treated by methyl hydrogen polysiloxane or silane coupling agent and the like; metallic soap-treated; fluorine-treated by perfluoroalkyl phosphate diethanolamine salt or perfluoroalkyl silane and the like; hydrophobilized-treated by dextrin fatty acid ester.

A liquid oil includes, for example, avocado oil, camellia oil, turtle oil, macadamia nut oil, corn oil, mink oil, olive oil, rape seed oil, yolk oil, sesame oil, persic oil, wheat embryo oil, sasanqua oil, castor oil, flaxseed oil, safflower oil, cottonseed oil, perilla oil, soybean oil, peanut oil, tea seed oil, kaya oil, rice bran oil, shinagiri oil, Japan tung oil, jojoba oil, embryo oil, triglycerin, etc.

A solid oil includes, for example, cocoa butter, coconut oil, equine tallow, hydrogenated coconut oil, palm oil, beef tallow, mutton tallow, hydrogenated beef oil, palm kernel oil, lard, bovine bone tallow, Japan kernel oil, hydrogenated oil, bovine leg tallow, Japan oil, hydrogenated castor oil, etc.

Waxes include, for example, beeswax, candelilla wax, cotton wax, carnauba wax, baybery wax, ibota wax, spermaceti wax, montan wax, rice wax, lanolin, kapok wax, lanolin acetate, liquid lanolin, sugarcane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether, etc.

Hydrocarbon oils include, for example, liquid paraffin, ozokerite, squalane, pristane, paraffin, ceresin, squalene, vaseline, microcrystalline wax, polyethylene wax, Fischer-Tropsch wax, etc.

Higher fatty acids include, for example, lauric acid, myristic acid, palmitic acid, stearic acid, behenic acid, oleic acid, undecylenic acid, tolic acid, linoleic acid, linolenic acid, eicosapentaenoic acid (EPA), docosahexaenoic acid (DHA), etc.

Higher alcohols include, for example, straight chain alcohols (e.g., lauryl alcohol, cetyl alcohol, stearyl alcohol, behenyl alcohol, myristyl alcohol, oleyl alcohol, cetostearyl alcohol, etc.); branched chain alcohols (e.g., monostearyl glycerin ether (batyl alcohol), 2-decyltetradecynol, lanolin alcohol, cholesterol, phytosterol, hexyldodecanol, octyldodecanol, etc.) and the like.

Synthesized ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethyl-hexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, 2-ethylhexyl succinate, triethyl citrate, polyoxethylene-polyoxypropylene random polymer methyl ether, etc.

Silicone oilsinclude, for example, chain polysiloxanes (e.g., dimethyl polysiloxane, methylphenylpolysiloxane, diphenylpolysiloxane, etc.); cyclic polysiloxanes (e.g., octamethylcyclotetrasiloxane, decamethylcyclopentasiloxane, dodecamethylcyclohexasiloxane, etc.), silicone resins which form three dimensional network structures, silicone gums, a wide variety of denatured polysiloxanes (amino-denatured polysiloxane, polyether-denatured polysiloxane, alkyl-denatured polysiloxane, fluorine-denatured polysiloxane, etc.) and the like.

Others include, for example, humectants such as polyethylene glycol, glycerin, 1 ,3-butyleneglycol, erythritol, sorbitol, xylitol, maltitol and the like; thickeners such as cellulose, hydroxyethylcellulose, hydroxypropylcellulose, methylhydroxypropylcellulose, methylcellulose, carboxymethylcellulose, quinceseed, carrageenan, pectin, mannan, curdlan, chondroitin sulfate, starch, galactan, dermatan sulfate, glycogen, acacia gum, heparan sulfate, hyaluronic acid, sodium hyaluronate, tragacanth gum, keratan sulfate, chondroitin, xanthan gum, mucoitin sulfate, hydroxyethyl guar gum, carboxymethyl guar gum, guar gum, dextran, kerato sulfate, locust bean gum, succinoglucan, caronic acid, chitin, chitosan, carboxymethylchitin and agar and the like; lower alcohols such as ethanol and the like; antioxidants such as butylhydroxytoluene, tocopherol, and phytin and the like; antibacterial agents such as benzoic acid, salicylic acid, sorbic acid, paraoxybenzoic acid alkyl ester, and hexachlorophene and the like; organic acids such as acyl sarcosine acid (e.g., sodium lauroyl sarcosine), glutathione, citric acid, malic acid, tartaric acid and lactic acid and the like; vitamin A and its derivatives, vitamin Bs such as vitamin B6 hydrochloride, vitamin B6 tripalmitate, vitamin B6 dioctanoate, vitamin B2 and its derivatives, vitamin B12, and vitamin B15 and its derivatives and the like, vitamin Cs such as ascorbic acid, ascorbic acid sulfate ester (salt), ascorbic acid phosphate ester (salt), and ascorbic acid dipalmitate and the like, vitamin E such as α-tocopherol, β-tocopherol, δ-tocopherol, vitamin E acetate and the like; vitamins such as vitamin Ds, vitamin H, pantothenic acid, and pantethine and the like; saponins such as nicotinamide, benzyl nicotinate, γ-orizanol, allantoin, glycyrrhizic acid (salt), glycyrrhetic acid and its derivatives, hinokitiol, bisabolol, eucalyptone, thymol, inositol, saikosaponin, ginseng saponin, Luffa cylindrica saponin, and soapberry saponin and the like; various drugs such as pantothenylethyl ether, ethinylestradiol, tranexamic acid, arbutin, cepharanthin, and placenta extract and the like; plant extracts such as Rumex japonicus, Clara, Nuphar Japonicum DC, orange, sage, Achillea alpina, Malva sylvestris, Swertia japonica, thyme, japanese angelica, bitter orange peel, birch, Field Horsetail, Luffa cylindrica, common horse-chestnut, Saxifragaceae, arnica, lily, sagebrush, paeoniae radix, aloe, Gardenia jasminoides, Sawara cypress, Hawthorn extract, Hypericum perforatum extract, iris-inextract, Gambir extract, ginkgo leaf extract, ibukijyakou extract, Foeniculum vulgare extract, Oolong tea extract, water-lily extract, Rosae Multiflorae Fructus extract, Plectranthus japonicus extract, Scutellariae Radix extract, Phellodendri Cortex extract, Lamium album extract, G. uralensis extract, Gardenia jasminoides extract, black tea extract, Tamarix chinensis Lour extract, Potentilla Erecta extract, rose extract, Luffa cylindrica extract, peppermint extract, rosemary extract, and royal jelly extract, pigment and the like; non-ionic active surfactants such as sorbitan monolaurate, sorbitan monopalmitate, sorbitan sesquioleate, sorbitan trioleate, polyoxyethylene sorbitan monolaurate, polyoxyethylene sorbitan monostearate, polyethylene glycol monooleate, polyoxyethylene alkyl ether, polyglycol diether, lauroyl diethanol amide, fatty acid isopropanol amide, maltitol hydroxy fatty acid ether, alkylated polysaccharide, alkyl glucoside, and sugar ester and the like; cationic surfactants such as stearyltrimethylammonium chloride, benzalkonium chloride, and laurylamine oxide and the like; anionic surfactants such as sodium palmitate, sodium laurate, sodium lauryl acid, potassium lauryl sulfate, alkylsulfuric acid triethanolamine ether, turkey-red oil, linear dodecylbenzene sulfate, polyoxyethylene hydrogenated castor oil maleic acid, and acylmethyl taurine and the like; ampholytic surfactants; neutralizers; antioxidants such as δ-tocopherol and butylhydroxytoluene and the like; and preservatives such as phenoxyethanol and paraben.

The above described ingredients are only illustrative and not limited to. Additionally, these ingredients may be mixed being combined appropriately according to description depending on the desired form.

Use of the dermatological external agent mixed with an anti-wrinkle agent of the invention enables prevention/amelioration of wrinkles, particularly fine wrinkles to provide the young and fresh skin.

In the method of the invention for preventing or ameliorating wrinkles, alkyl glucosides or salts thereof may be applied in any forms singly or in combination with any other ingredients as long as they can be applied to the skin and can attain the purpose of the invention. In addition, the location of the skin where they are applied to is not limited, including the skin of all over the body surface including the scalp. The method of the invention is used usually as a cosmetic method.

In addition, Use of the ADAM inhibitor consisting of an alkyl glucoside or a salt thereof is not limited to prevention or amelioration of wrinkles as described above and includes any uses which can exert the effect by inhibiting the ADAM activity. For example, they may be used for treatment or prevention of different diseases adversely affected by growth or differentiation due to the activation of the ADAM.

The ADAM inhibitor of the invention may be used as a drug intended to prevent or ameliorate the conditions resulted from increase of the ADAM activity.

In order to formulate the ADAM inhibitor of the invention in such uses, for example, external agents, powders, granules, ampules, injections, isotonic solutions and the like are prepared by ordinary methods. In the case of preparing oral solid preparations, excipients and if needed binders, wetting agents, disintegrators, surfactants, lubricants, dispersing agents, flavors and the like are added followed by preparation as tablets, coated tablets, granules, capsules and the like according to conventional methods.

The excipients used include, for example, lactose, glucose, sorbitol, cornstarch and mannitol, etc.; the binders include, for example, polyvinyl alcohol, polyvinyl ether, ethylcellulose, acacia gum, gelatin, hydroxypropylcellulose and polyvinylpyrrolidone, etc.; the disintegrators include calcium carbonate, calcium citrate, dextrin, starch and gelatin powder etc.; the lubricants include calcium carbonate, calcium citrate, talc, polyethyleneglycol, etc.; the coloring agents include cocoa powder, mint flavoring acid and mint oil, etc. These tablets and granules may be coated properly if needed by sugar, gelatin and others.

In the case of preparing the injections, pH adjusting agents, buffers, surfactants, solubilizers, solvents, stabilizers, preservatives and the like are added if needed and prepared as subcutaneous, intramuscular or intravenous

injections.

Examples

Examples will now be given to describe the present invention in more detail. The invention is not intended to be limited to them. The combination amounts herein are % by mass.

1. Exploring a new ADAM inhibitor

For ADAM inhibiting effect, the TNF-α which is cut out to be released from the membrane of the U937 cell derived from the human lymphoma through the action of the ADAM was used as an index and the inhibiting effect on the TNF-α release was assessed. A new ADAM inhibitor was explored by screening different compounds according to the present method.

The U937 cells derived from the human lymphoma were seeded to the 48-well plate adjusting the number of the cells to $1\times10^5$ cells/0.25 ml and cultured at 37°C overnight. The medium which contained 30 μg/ml of alkyl glucoside was added and incubated at 37°C for 30 min. to be pretreated. 10 nM PMA (phorbol ester: 12-O-tetradecanoylphorbol-acetate; Sigma P8139) was then added and cultured at 37°C for 6 hours. After completion of the treatment, the supernatant of the culture was collected, centrifuged and the cell-free supernatant was cryopreserved at -20°C. The assay of the released hTNF-α was performed using the hTNF-α Duo-Set ELISA kit from R & D Ltd. Each supernatant of the culture was diluted 5 folds with the medium, then 100 μl each was added to the 96-well plate and measured according to the protocol of R & D Ltd. The absorbance at 450-570 nm of each well was measured by the measurement kit and concentrations of the samples were obtained from the standard curve. The inhibition rates were calculated according to the following equation wherein A0 is the absorbance of 0% inhibiting control (the medium containing only PMA), A100 is the absorbance of 100% inhibiting control (only the medium) and AS is the absorbance of the sample.

$$\text{The inhibition rate (\%)} = (A0 - AS) / (A0 - A100) \times 100$$

Consequently, a high inhibitory effect on HB-EGF release was noted for the inventive alkyl glucoside suggesting that it inhibits the ADAM activity. The release inhibion rate of each alkyl glucoside is shown in Table 1. For comparison, the glucoside compounds represented by formulas (A)-(E) were also tested to obtain their release inhibition rates. The results are shown in Table 1. It is found that existence of the alkyl group is essential because there is no effect noted in the glucoside compounds shown in the formulae (A)-(E) from the Table 1.

[Formula 4]

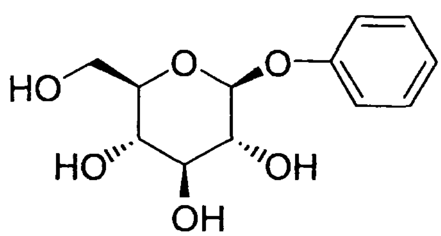

(A)

[Formula 5]

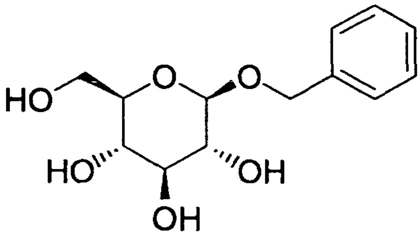

(B)

[Formula 6]

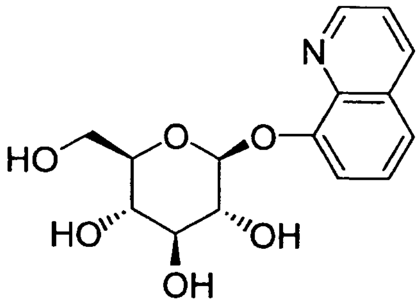

(C)

[Formula 7]

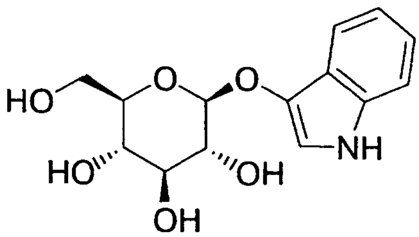

(D)

[Formula 8]

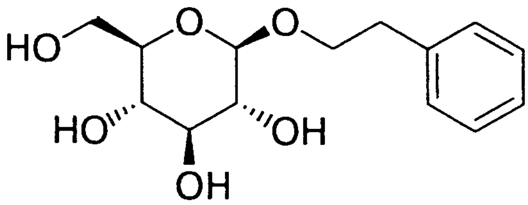

(E)

[Table 1]

| Tested substance | Inhibition rate (%) |
|---|---|
| | Concentration |
| | 30 μg/mL |
| Decyl β-D-glucoside | 56 |
| Undecyl β-D-glucoside | 98 |
| Dodecyl β-D-glucoside | 88 |
| Dodecyl α-D-glucoside | 91 |
| Tetradecyl β-D-glucoside | 109 |
| Hexadecyl β-D-glucoside | 76 |
| Octadecyl β-D-glucoside | 62 |
| Glucoside compound (A) | 9.5 |
| Glucoside compound (B) | 3.4 |
| Glucoside compound (C) | 4.8 |
| Glucoside compound (D) | -0.5 |
| Glucoside compound (E) | -51.83 |

2. Assessment of wrinkle-ameliorating effect of the alkyl glucosides

Then, the wrinkle-ameliorating effects of dodecyl β-D-glucoside (GC12), tetradecyl β-D-glucoside (GC14), and hexadecyl β-D-glucoside (GC16) amongst the new ADAM-inhibiting compounds were examined using a barrier-destructed wrinkle mouse model.

The tape stripping was performed on the left back of the hairless mice (HR-1, 6 week old male, Hosino experimental animals) 3 times a week for 4 consecutive weeks so that the trans epithelial water loss (measured using a measuring device of water loss MEECO; Meeco Ltd, USA) was 4-8 mg/cm$^2$/h. Every time immediately after procedures of tape stripping, 100 μl each of the solutions of dodecyl β-D-glucoside, tetradecyl β-D-glucoside, and hexadecyl β-D-glucoside dissolved in the 50% ethanol aqueous solution so that they were 1 % were applied on the left back of the mice, and as the negative control, a 50% ethanol aqueous solution (vehicle) were similarly applied. Number n was set as 6 to 7 for each group.

States of the wrinkle development after 4 weeks were scored by visual judgement. The assessment criteria of the wrinkle development state was set as "no wrinkle; 0", "thin wrinkle; 1 ", "clear wrinkle; 2", "deep wrinkle; 3", and the states were scored to the 0.5. As the score is greater, it shows the wrinkle is deeper. The average value and standard

deviation of each group is calculated and the result is shown in Figure 2.

Obviously from the figure 2, in contrast to the Vehicle-applied group with the average score of 1.24, those of the groups which were applied dodecyl β-D-glucoside, tetradecyl β-D-glucoside and hexadecyl β-D-glucoside were significantly lower with the scores of 0.58, 0.63 and 0.89, respectively indicating their marked wrinkle-inhibiting effect.

Further, the trans epithelial water loss (TEWL) at the backs of the mice before starting the test and at 4 weeks after starting was also examined. The TEWL values at the left backs on which dodecyl β-D-glucoside, tetradecyl β-D-glucoside, and hexadecyl β-D-glucoside or 50% ethanol aqueous solution (vehicle) were applied after tape stripping procedure as well as the values at the right backs on which nothing was applied were measured by the Vapometer (Technologies Ltd, Kuopio, Finland) two times, respectively. The ratio of the TEWL value for the left side based on the TEWL value of the right side for each individual (the relative TEWL (%)) was obtained and the average value for each group was calculated. As the percentage value is higher, it shows the roughness of the skin is increased. The results are shown in Figure 3.

Obviously from Fig. 3, in contrast to the vehicle-applied group which increased the TEWL to 186%, the increase was stayed to 141 % for the dodecyl β-D-glucoside-applied group, 130% for the tetradecyl β-D-glucoside group and 164% for the β-D-glucoside-applied group, showing that dodecyl β-D-giucoside and tetradecyl β-D-glucoside significantly inhibited the increase of the TEWL caused by the tape stripping. The rate of the TEWL increase for hexadecyl β-D-glucoside was also lower compared to the vehicle.

Using the similar method, the wrinkle-inhibiting effect of octadecyl β-D-glucoside (GC18) was also examined. Obviously from Fig. 4, by visual judgement, the average value of the wrinkle score for the vehicle-applied group was 1.55, whereas the wrinkle score for the octadecyl β-D-glucoside-applied group was significantly inhibited to 0.54. In addition, as shown in Fig. 5, the TEWL was increased to 208% for the vehicle-applied group, whereas the increase of the TEWL stayed to 124% for the octadecyl β-D-glucoside-applied group showing that octadecyl β-D-glucoside significantly inhibited the increase of the TEWL caused by the tape stripping.

From these results, it was suggested that dodecyl β-D-glucoside, tetradecyl β-D-glucoside, hexadecyl β-D-glucoside, and octadecyl β-D-glucoside may inhibit epidermal growth factors (HB-EGF etc.) released by the ADAM together with TNF-α through inhibition the activated ADAM leading to control of skin proliferation and thickening resulting in prevention or amelioration of development of wrinkles.

3. Assessment of ameliorating effect of alkyl glucoside on fine wrinkles around the human eyes

Then, the wrinkle ameliorating effect on human of dodecyl β-D-glucoside (GC12) among the new ADAM-inhibiting compounds which had showed the high wrinkle ameliorating effect in the barrier-destructed wrinkle mouse was examined by the half phase double blind test.

An aqueous solution of 0.44% dodecyl β-D-glucoside/30% ethanol was successively applied to 16 adult male humans in the 30-40th's (the average age of 42.2) for two months. The dosage was set as 30 μl per application. The solution of 0.44% dodecyl β-D-glucoside/30% ethanol was applied to the lateral angle of and under one eye and 30% ethanol aqueous solution as the vehicle was applied to those of the other eye two times a day.

To examine the state of the skin around the eyes before the application and after the application, it was acclimated in a steady temperature and humidity room (23.5°C, 45% humidity) after washing the face and then photo taking of the appearance around the eyes (Nikon digital single lens reflex camera D-100) and the water content measurement of the horny layer (Corneometer, Courage+Khazaka electronic GmbH, Germany) were performed before starting the test and after starting the successive application for two months.

Fig. 6 shows the change of the water content of the corneal layer over time. The rate of the water content value of the corneal layer of the eye, when the value is 100, after successive application for two months based on the water content value of the horny layer of each subject before starting the test (relative water content of the horny layer (%)) was obtained to calculate the average value for each group. The water content of the corneal layer reduced to 95% of the value observed before start for the vehicle-applied group, whereas reduction of the water content of the corneal layer was controlled for the dodecyl β-D-glucoside group.

Then, the visual appreciation judgement was performed by three judging persons. By consultation of three judging persons using the photographs of the site under the eye of each 16 adult male human subject in the 30-40th's taken before starting and two months after starting, the case wherein the dodecyl β-D-glucoside-applied side showed obviously reduced number of the fine wrinkles or shallowed fine wrinkles compared to the vehicle-applied side was set as " ameliorated", the case wherein distinct difference was not noted between the both sides was set as "unchanged", and the case wherein the dodecyl β-D-glucoside-applied side showed obviously increased number of the fine wrinkles or deepened the fine wrinkles compared to the vehicle-applied side was set as "deteriorated".

Upon summing up the number of the subjects for each category followed by performing the Wilcoxon signed rank sum test, it was revealed that dodecyl β-D-glucoside significantly ameliorates the fine wrinkles under the eye as shown in Table 2.

[Table 2]

| | Ameliorated | Deteriorated | Unchanged | Wilcoxon test |
|---|---|---|---|---|
| Number of subjects | 10 | 2 | 4 | 0.0386 |

Examples of preparations of the dermatological external agent containing an alkyl glucoside derivative or a salt thereof of the invention will be shown below, but the invention is not to be limited to them. All combination amounts are represented by % by mass based on the total weight of the external agent.

Preparation Example 1: emulsion

| (Formulation) | % by mass |
|---|---|
| Stearic acid | 2.5 |
| Cetyl alcohol | 1.5 |
| Vaseline | 5.0 |
| Liquid paraffin | 5.0 |
| Polyoxyethylene(10mol) monooleate ester | 2.0 |
| Polyethylene glycol 1500 | 3.0 |
| Triethanolamine | 1.0 |
| Carboxy vinyl polymer | 0.05 |
| Decyl β-D-glucoside | 2.0 |
| Sodium bisulfite | 0.01 |
| Ethylparaben | 0.2 |
| Flavor | q.s. |
| Purified water | balance |

(Procedure)

Carboxy vinyl polymer is dissolved in a little amount of the purified water (A phase). Polyethylene glycol 1500, triethanolamine and decyl β-D-glucoside are added to the remaining purified water, and heated, dissolved then maintained at 70°C (aqueous phase). Other ingredients are mixed, heated and melted then maintained at 70°C (oil phase). The oil phase is added to the aqueous phase to preemulsify, then A phase is added to emulsify homogeneously. After emulsification, it is cooled to 30°C while mixing well to obtain an emulsion.

Preparation Example 2: lotion

| (Formulation) | % by mass |
|---|---|
| Ethanol | 5.0 |
| Carboxy vinyl polymer | 0.3 |
| Polyoxyethylene(15 mol)oleyl ether | 0.8 |
| 1,3-Butylene glycol | 5.0 |
| Undecyl β-D-glucoside | 2.0 |
| Citric acid | 0.03 |
| Sodium citrate | 0.07 |
| Methylparaben | 0.1 |
| Purified water | balance |

(Procedure)

Citric acid, sodium citrate and undecyl β-D-glucoside were dissolved in the purified water to make an aqueous phase. On the other hand, other ingredients stirred and dissolved were added to the aqueous phase and were made homogeneous to obtain a lotion.

| Preparation Example 3: cream | |
|---|---|
| (Formulation) | % by mass |
| Stearic acid | 2.0 |
| Stearyl alcohol | 7.0 |
| Hydrous lanolin | 2.0 |
| 2-Octyldodecyl alcohol | 6.0 |
| Polyoxyethylene(25 mol)cetyl alcohol ether | 3.0 |
| Glycerin monostearate ester | 2.0 |
| Dodecyl β-D-glucoside | 2.0 |
| Dodecyl α-D-glucoside | 2.0 |
| 1,3-Butylene glycol | 5.0 |
| Trisodium edetate | 0.1 |
| Sodium glycyrrhetinate | 0.1 |
| Vitamin E acetate | 0.3 |
| Ethylparaben | 0.3 |
| Purified water | balance |

(Procedure)

Dodecyl β-D-glucoside, dodecyl α-D-glucoside, 1,3-butylene glycol, trisodium edetate and sodium glycyrrhetinate were added to the purified water and maintained at 70°C to make an aqueous phase. On the other hand, other ingredients were mixed, heated and melted then maintained at 70°C to make an oil phase. The oil phase was added to the aqueous phase to preemulsify, then emusified homogenously with a homomixer followed by cooling to 30°C to obtain a cream.

| Preparation Example 4: emulsion | |
|---|---|
| (Formulation) | % by mass |
| Dimethyl polysiloxane | 3.0 |
| Decamethylcyclopenta siloxane | 4.0 |
| Ethanol | 5.0 |
| Glycerin | 6.0 |
| 1,3-Butylene glycol | 5.0 |
| Tetradecyl β-D-glucoside | 0.5 |
| Hexadecyl β-D-glucoside | 0.5 |
| Polyoxyethylene methyl glucoside | 3.0 |
| Sunflower oil | 1.0 |
| Squalene | 2.0 |
| Potassium hydroxide | 0.1 |
| Sodium hexametaphosphate | 0.05 |
| Hydoxypropyl-β-cyclodextrin | 0.1 |
| Potassium 4-methoxysalicilate | 1.0 |
| Dipotassium glycyrrhizinate | 0.05 |
| Loquat leaf extract | 0.1 |
| Sodium L-glutamate | 0.05 |
| Fennel extract | 0.1 |
| Yeast extract | 0.1 |
| Lavender extract | 0.1 |
| Rehrnannia root extract | 0.1 |
| Dimorphorinopyridazinone | 0.1 |
| Xanthan gum | 0.1 |

(continued)

| Preparation Example 4: emulsion | |
|---|---|
| (Formulation) | % by mass |
| Carboxy vinyl polymer | 0.1 |
| Alkyl acrylate/methacrylate copolymer(PemulenTR-1) | 0.1 |
| Colcothar | q.s. |
| Yellow oxide | q.s. |
| Paraben | q.s. |
| Purified water | balance |

| Preparation Example 5: emulsion | |
|---|---|
| (Formulation) | % by mass |
| Dimethyl polysiloxane | 3.0 |
| Decamethylcyclopentasiloxane | 4.0 |
| Ethanol | 5.0 |
| Glycerin | 6.0 |
| 1,3-Butylene glycol | 5.0 |
| Octadecyl β-D-glucoside | 1.0 |
| Polyoxyethylene methyl glucoside | 3.0 |
| Sunflower oil | 1.0 |
| Squalene | 2.0 |
| Potassium hydroxide | 0.1 |
| Sodium hexametaphosphate | 0.05 |
| Hydoxypropyl-β-cyclodextrin | 0.1 |
| Potassium 4-methoxysalicilate | 1.0 |
| Dipotassium glycyrrhizinate | 0.05 |
| Loquat leaf extract | 0.1 |
| Sodium L-glutamate | 0.05 |
| Fennel extract | 0.1 |
| Yeast extract | 0.1 |
| Lavender extract | 0.1 |
| Rehrnannia root extract | 0.1 |
| Dimorphorinopyridazinone | 0.1 |
| Xanthan gum | 0.1 |
| Carboxy vinyl polymer | 0.1 |
| Alkyl acrylate/methacrylate copolymer (PemulenTR-1) | 0.1 |
| Colcothar | q.s. |
| Yellow oxide | q.s. |
| Paraben | q.s. |
| Purified water | balance |

| Preparation Example 6: emulsion | |
|---|---|
| (Formulation) | % by mass |
| Dimethyl polysiloxane | 2.0 |
| Behenyl alcohol | 1.0 |
| Batyl alcohol | 0.5 |
| Glycerin | 5.0 |
| 1,3-Butylene glycol | 7.0 |

(continued)

| Preparation Example 6: emulsion | |
| --- | --- |
| (Formulation) | % by mass |
| Dodecyl β-D-glucoside | 0.5 |
| Tetradecyl β-D-glucoside | 1.0 |
| Tranexamic acid methylamide hydrochloride | 0.7 |
| Erythritol | 2.0 |
| Hydrogenated oil | 3.0 |
| Squalene | 6.0 |
| Pentaerythrit tetra 2-ethylhexanoate | 2.0 |
| Polyoxyethylene glyceryl isostearate | 1.0 |
| Polyoxyethylene glycerin monostearate | 1.0 |
| Potassium hydroxide | q.s. |
| Sodium hexametaphosphate | 0.05 |
| Phenoxyethanol | q.s. |
| Carboxy vinyl polymer | 0.1 |
| Purified water | balance |

| Preparation Example 7: emulsion | |
| --- | --- |
| (Formulation) | % by mass |
| Dimethyl polysiloxane | 2.0 |
| Behenyl alcohol | 1.0 |
| Batyl alcohol | 0.5 |
| Glycerin | 5.0 |
| 1,3-Butylene glycol | 7.0 |
| Hexadecyl β-D-glucoside | 1.0 |
| Tranexamic acid methylamide hydrochloride | 0.7 |
| Erythritol | 2.0 |
| Hydrogenated oil | 3.0 |
| Squalene | 6.0 |
| Pentaerythrit tetra 2-ethylhexanoate | 2.0 |
| Polyoxyethylene glyceryl isostearate | 1.0 |
| Monostearic acid polyoxyethylene glycerin | 1.0 |
| Potassium hydroxide | q.s. |
| Sodium hexametaphosphate | 0.05 |
| Phenoxyethanol | q.s. |
| Carboxy vinyl polymer | 0.1 |
| Purified water | balance |

| Preparation Example 8: emulsion | |
| --- | --- |
| (Formulation) | % by mass |
| Liquid paraffin | 7.0 |
| Vaseline | 3.0 |
| Decamethylcyclopentasiloxane | 2.0 |
| Behenyl alcohol | 0.5 |
| Glycerin | 5.0 |
| Dipropylene glycol | 7.0 |
| Octadecyl β-D-glucoside | 1.0 |

(continued)

| Preparation Example 8: emulsion | |
|---|---|
| (Formulation) | % by mass |
| Dodecyl α-D-glucoside | 1.0 |
| Polyethylene glycol 1500 | 2.0 |
| Jojoba oil | 1.0 |
| Isostearic acid | 0.5 |
| Stearic acid | 0.5 |
| Behenic acid | 0.5 |
| Pentaerythrit tetra 2-ethylhexanoate | 3.0 |
| Cetyl 2- ethylhexanoate | 3.0 |
| Glyceryl monostearate | 1.0 |
| Monostearic acid polyoxyethylene glycerin | 1.0 |
| Potassium hydroxide | 0.1 |
| Sodium hexametaphosphate | 0.05 |
| Stearyl glycyrrhetinate | 0.05 |
| L-arginine hydrochloride | 0.1 |
| Royal jelly extract | 0.1 |
| Yeast extract | 0.1 |
| Curcuma extract | 0.1 |
| Tocopherol acetate | 0.1 |
| Acetylated sodium hyaluronate | 0.1 |
| Trisodium edetate | 0.05 |
| 4-t-Butyl-4'-methoxydibenzoylmethan | 0.1 |
| 2-Ethylhexyl Para-methoxycinnamate | 0.1 |
| Carboxy vinyl polymer | 0.15 |
| Paraben | q.s. |
| Purified water | balance |
| Flavor | q.s. |

| Preparation Example 9: emulsion | |
|---|---|
| (Formulation) | % by mass |
| Vaseline | 5.0 |
| Behenyl alcohol | 0.5 |
| Batyl alcohol | 0.5 |
| Glycerin | 7.0 |
| 1,3-Butylene glycol | 7.0 |
| Dodecyl β-D-glucoside | 1.0 |
| Hexadecyl β-D-glucoside | 1.0 |
| 1,2-pentanediol | 1.0 |
| Xylit | 3.0 |
| Polyethylene glycol 20000 | 2.0 |
| Hydrogenated oil | 2.0 |
| Jojoba oil | 2.0 |
| Squalene | 5.0 |
| Isostearic acid | 0.5 |
| Pentaerythrit tetra2-ethylhexanoate | 2.0 |
| Polyoxyethylene hydrogenated castor oil | 0.5 |
| Lauryldimethylaminoacetic acid betaine | 0.4 |
| Potassium hydroxide | q.s. |

(continued)

| Preparation Example 9: emulsion | |
|---|---|
| (Formulation) | % by mass |
| Sodium pyrosulfite | 0.01 |
| Sodium hexametaphosphate | 0.05 |
| Dipotassium glycyrrhizinate | 0.05 |
| Trimethylglycine | 3.0 |
| Arbutin | 3.0 |
| Pyrola japonica extract | 0.1 |
| Deadnettle extract | 0.1 |
| Lempuyang extract | 0.1 |
| Yeast extract | 0.1 |
| Tocopherol acetate | 0.1 |
| Thiotaurine | 0.1 |
| Clara extract | 0.1 |
| Colcothar | q.s. |
| Qince seed extract | 0.1 |
| Carboxy vinyl polymer | 0.2 |
| Phenoxyethanol | q.s. |
| Purified water | balance |

| Preparation Example 10: emulsion | |
|---|---|
| (Formulation) | % by mass |
| Vaseline | 5.0 |
| Dimethyl polysiloxane | 2.0 |
| Behenyl alcohol | 0.6 |
| Batyl alcohol | 0.5 |
| Dipropylene glycol | 2.0 |
| 1,3-Butylene glycol | 4.0 |
| Dodecyl β-D-glucoside | 10.0 |
| Octadecyl β-D-glucoside | 3.0 |
| Xylit | 1.0 |
| Polyethylene glycol 1500 | 1.0 |
| Squalene | 5.0 |
| Glyceryl Tri2-ethylhexanoate | 2.0 |
| Retinol acetate | 0.03 |
| Retinol (1.5 million unit) | 0.01 |
| Polyoxyethylene hydrogenated castor oil | 0.5 |
| Tranexamic acid methylamide hydrochloride | 0.7 |
| Dipotassium glycyrrhizinate | 0.1 |
| 2-O-ethyl-L-ascorbic acid | 0.1 |
| Yeast extract | 0.1 |
| Gambir extract | 0.1 |
| Peony root extract | 0.1 |
| Belamcanda extract | 0.1 |
| Trisodium HEDTA | 0.05 |
| Xanthan gum | 0.1 |
| Carboxy vinyl polymer | 0.15 |
| Purified water | balance |
| Flavor | q.s. |

| Preparation Example 11: lotion | |
|---|---|
| (Formulation) | % by mass |
| Ethylalcohol | 5.0 |
| Glycerin | 1.0 |
| 1,3-Butylene glycol | 5.0 |
| Dodecyl β-D-glucoside | 3.0 |
| Decyl β-D-glucoside | 10.0 |
| Polyoxyethylene polyoxypropylene decyl tetradecyl ether | 0.2 |
| Sodium hexametaphosphate | 0.03 |
| Trimethylglycine | 1.0 |
| Sodium polyaspartate | 0.1 |
| α-Tocopherol 2-L-ascorbate diesterpotassium phosphate | 0.1 |
| 2-O-ethyl-L-ascorbic acid | 0.1 |
| Green tea extract | 0.1 |
| Beech extract | 0.1 |
| Peach kernel | 0.1 |
| Peppermint extract | 0.1 |
| Iris root extract | 0.1 |
| Trisodium HEDTA | 0.1 |
| Carboxy vinyl polymer | 0.05 |
| Potassium hydroxide | 0.02 |
| Phenoxyethanol | q.s. |
| Purified water | balance |
| Flavor | q.s. |

| Preparation Example 12: lotion | |
|---|---|
| (Formulation) | % by mass |
| Glycerin | 2.0 |
| 1,3-Butylene glycol | 4.0 |
| Undecyl β-D-glucoside | 1.0 |
| Dodecyl β-D-glucoside | 1.0 |
| Erythritol | 1.0 |
| Polyoxyethylene methylglucoside | 1.0 |
| Polyoxyethylene hydrogenated castor oil | 0.5 |
| L-ascorbic acid 2-glucoside | 2.0 |
| Xylitol | 0.1 |
| Citric acid | 0.02 |
| Sodium citrate | 0.08 |
| Phenoxyethanol | q.s. |
| N-coconut oil fatty acid acyl L-arginine ethyl/DL-pyrrolidone carboxylic acid | 0.1 |
| Purified water | balance |

| Preparation Example 13: lotion | |
|---|---|
| (Formulation) | % by mass |
| Glycerin | 2.0 |
| 1,3-Butylene glycol | 4.0 |

(continued)

| Preparation Example 13: lotion | |
|---|---|
| (Formulation) | % by mass |
| Dodecyl α-D-glucoside | 1.0 |
| Tetradecyl β-D-glucoside | 1.0 |
| Erythritol | 1.0 |
| Polyoxyethylene methylglucoside | 1.0 |
| Polyoxyethylene hydrogenated castor oil | 0.5 |
| L-ascorbic acid 2-glucoside | 2.0 |
| Xylitol | 0.1 |
| Citric acid | 0.02 |
| Sodium citrate | 0.08 |
| Phenoxyethanol | q.s. |
| N-coconut oil fatty acid acyl L-arginine ethyl/DL-pyrrolidone carboxylic acid | 0.1 |
| Purified water | balance |

| Preparation Example 14: lotion | |
|---|---|
| (Formulation) | % by mass |
| Ethanol | 10.0 |
| Dipropylene glycol | 1.0 |
| Hexadecyl β-D-glucoside | 1.0 |
| Octadecyl β-D-glucoside | 1.0 |
| Polyethylene glycol 1000 | 1.0 |
| Polyoxyethylene methylglucoside | 1.0 |
| Jojoba oil | 0.01 |
| Glyceryl tri2-ethylhexanate | 0.1 |
| Polyoxyethylene hydrogenated castor oil | 0.2 |
| Polyglyceryl diisostearate | 0.15 |
| Sodium N-stearoiyl-L-glutamate | 0.1 |
| Citric acid | 0.05 |
| Sodium citrate | 0.2 |
| Potassium hydroxide | 0.4 |
| Dipotassium glycyrrhizinate | 0.1 |
| Arginine hydrochloride | 0.1 |
| L-ascorbic acid 2-glucoside | 2.0 |
| Scutellaria extract | 0.1 |
| Saxifraga stolonifera extract | 0.1 |
| Deadnettle extract | 0.1 |
| Thyme extract | 0.1 |
| Origanum majorana extract | 0.1 |
| Tranexamic acid | 1.0 |
| Trisodium edetate | 0.05 |
| 2-Ethylhexyl para-methoxycinnamate | 0.01 |
| Dibutylhydroxytoluene | q.s. |
| Paraben | q.s. |
| Ocean deep water | 3.0 |
| Purified water | balance |
| Flavor | q.s. |

| Preparation Example 15: lotion | |
|---|---|
| (Formulation) | % by mass |
| Dimethyl polysiloxane | 1.0 |
| Ethanol | 3.0 |
| Behenyl alcohol | 0.3 |
| Glycerin | 5.0 |
| Dipropylene glycol | 5.0 |
| Dodecyl β-D-glucoside | 0.1 |
| Tetradecyl β-D-glucoside | 0.1 |
| Erythritol | 1.0 |
| Polyethylene glycol 4000 | 1.0 |
| Squalene | 0.4 |
| Cetyl 2-ethylhexanoate | 0.1 |
| Sodium N-stearoyl-L-glutamate | 0.2 |
| Magnesium chloride | 0.1 |
| Arginine chloride | 0.1 |
| Hypotaurine | 0.1 |
| Trisodium edetate | 0.1 |
| Paraben | q.s. |
| Purified water | balance |
| Flavor | q.s. |

| Preparation Example 16: lotion | |
|---|---|
| (Formulation) | % by mass |
| Ethanol | 40.0 |
| Dipropylene glycol | 1.0 |
| Undecyl β-D-glucoside | 0.1 |
| Dodecyl β-D-glucoside | 0.1 |
| Polyoxyethylene polyoxypropylene decyl tetradecyl ether | 0.1 |
| Anhydrous silicic acid | 1.0 |
| Salicylic acid | 0.1 |
| Sodium citrate | 0.2 |
| Zinc paraphenolsulfonate | 0.2 |
| Dipotassium glycyrrhizinate | 0.1 |
| Pyridoxine hydrochloride | 0.1 |
| L-Serine | 0.1 |
| L-Menthol | 0.05 |
| Trisodium HEDTA | 0.05 |
| Powdered cellulose | 1.0 |
| Bentonite | 0.8 |
| Purified water | balance |

| Preparation Example 17: lotion | |
|---|---|
| (Formulation) | % by mass |
| Ethanol | 40.0 |
| Dipropylene glycol | 1.0 |
| Dodecyl β-D-glucoside | 0.1 |

(continued)

| Preparation Example 17: lotion | |
|---|---|
| (Formulation) | % by mass |
| Polyoxyethylene polyoxypropylene decyl tetradecyl ether | 0.1 |
| Anhydrous silicic acid | 1.0 |
| Salicylic acid | 0.1 |
| Sodium citrate | 0.2 |
| Zinc paraphenolsulfonate | 0.2 |
| Dipotassium glycyrrhizinate | 0.1 |
| Pyridoxine hydrochloride | 0.1 |
| L-Serine | 0.1 |
| L-Menthol | 0.05 |
| Trisodium HEDTA | 0.05 |
| Powdered cellulose | 1.0 |
| Bentonite | 0.8 |
| Purified water | balance |

| Preparation Example 18: gel | |
|---|---|
| (Formulation) | % by mass |
| Dimethyl polysiloxane | 5.0 |
| Glycerin | 2.0 |
| 1,3-Butylene glycol | 5.0 |
| Dodecyl β-D-glucoside | 0.1 |
| Dodecyl α-D-glucoside | 0.1 |
| Polyethylene glycol 1500 | 3.0 |
| Polyethylene glycol 20000 | 3.0 |
| Cetyl octanoate | 3.0 |
| Citric acid | 0.01 |
| Sodium citrate | 0.1 |
| Sodium hexametaphosphate | 0.1 |
| Dipotassium glycyrrhizinate | 0.1 |
| L-ascorbic acid 2-glucoside | 2.0 |
| Magnesium L-ascorbate phosphate | 0.1 |
| Tocopherol acetate | 0.1 |
| Scutellaria extract | 0.1 |
| Thime extract | 0.1 |
| Strawberry geranium extract | 0.1 |
| Trisodium edetate | 0.1 |
| Xanthan gum | 0.3 |
| Alkyl acrylate/methacrylate copolymer(PemulenTR-2) | 0.05 |
| Powdered agar | 1.5 |
| Phenoxyethanol | q.s. |
| Dibutylhydrokytoluene | q.s. |
| Purified water | balance |

| Preparation Example 19: pack | |
|---|---|
| (Formulation) | % by mass |
| Ethanol | 10.0 |

(continued)

| Preparation Example 19: pack | |
|---|---|
| (Formulation) | % by mass |
| 1,3-Butylene glycol | 6.0 |
| Dodecyl β-D-glucoside | 1.0 |
| Tetradecyl β-D-glucoside | 1.0 |
| Polyethylene glycol 4000 | 2.0 |
| Olive oil | 1.0 |
| Macadamia nuts oil | 1.0 |
| Phytosteryl hydroxystearate | 0.05 |
| Lactic acid | 0.05 |
| Sodium lactate | 0.1 |
| L- Ascorbic acid 2 sodium sulfate | 0.1 |
| α-Tocopherol 2-L-ascorbate dipotassium phosphate | 0.1 |
| Vitamin E acetate | 0.1 |
| Fish collagen | 0.1 |
| Sodium chondroitin sulfate | 0.1 |
| Sodium carboxymethylcellulose | 0.2 |
| Polyvinyl alcohol | 12.0 |
| Para-oxybenzoic acid ester | q.s. |
| Purified water | balance |
| Flavor | q.s. |

| Preparation Example 20: pack | |
|---|---|
| (Formulation) | % by mass |
| Ethanol | 3.0 |
| Glycerin | 5.0 |
| 1,3-Butylene glycol | 6.0 |
| Hexadecyl β-D-glucoside | 1.0 |
| Octadecyl β-D-glucoside | 1.0 |
| Polyethylene glycol 1500 | 5.0 |
| Polyoxyethylenemethylglucoside | 2.0 |
| Glyceryl tri2-ethylhexanoate | 1.0 |
| Sodium hexametaphosphate | 0.05 |
| Hydroxypropyl-β-cyclodextrin | 0.1 |
| Dipotassium glycyrrhizinate | 0.1 |
| Loquat leaf extract | 0.1 |
| Sodium L-glutamate | 0.1 |
| Foeniculum vulgare extract | 0.1 |
| Hamamelis japonica extract | 0.1 |
| Phellodendron bark extract | 0.1 |
| Rehmannia glutinosa extract | 0.1 |
| Eucalyptus oil | 0.05 |
| Dimorpholinopyridazinone | 0.1 |
| Xanthan gum | 0.05 |
| Carboxy vinyl polymer | 0.5 |
| Acrylic acid/alkyl methacrylate copolymer (Pemulen | TR-1) 0.05 |
| Potassium hydroxide | 0.05 |
| Phenoxyethanol | q.s. |
| Purified water | balance |

| Preparation Example 21: pack | |
|---|---|
| (Formulation) | % by mass |
| Ethanol | 3.0 |
| Glycerin | 5.0 |
| 1,3-Butylene glycol | 6.0 |
| Dodecyl β-D-glucoside | 1.0 |
| Polyethylene glycol 1500 | 5.0 |
| Polyoxyethylenemethylglucoside | 2.0 |
| Glyceryl tri2-ethylhexanoate | 1.0 |
| Sodium hexametaphosphate | 0.05 |
| Hydroxypropyl-β-cyclodextrin | 0.1 |
| Dipotassium glycyrrhizinate | 0.1 |
| Loquat leaf extract | 0.1 |
| Sodium L-glutamate | 0.1 |
| Foeniculum vulgare extract | 0.1 |
| Hamamelis japonica extract | 0.1 |
| Phellodendron bark extract | 0.1 |
| Rehmannia glutinosa extract | 0.1 |
| Eucalyptus oil | 0.05 |
| Dimorpholinopyridazinone | 0.1 |
| Xanthan gum | 0.05 |
| Carboxy vinyl polymer | 0.5 |
| Acrylic acid/alkyl methacrylate copolymer (Pemulen | TR-1) 0.05 |
| Potassium hydroxide | 0.05 |
| Phenoxyethanol | q.s. |
| Purified water | balance |

| Preparation Example 22: cream | |
|---|---|
| (Formulation) | % by mass |
| Liquid paraffin | 3.0 |
| Vaseline | 1.0 |
| Dimethylpolysiloxane | 1.0 |
| Stearyl alcohol | 1.8 |
| Behenyl alcohol | 1.6 |
| Glycerin | 8.0 |
| Dipropylene glycol | 5.0 |
| Dodecyl β-D-glucoside | 5.0 |
| Octadecyl β-D-glucoside | 5.0 |
| Macadamia nuts oil | 2.0 |
| Hydrogenated oil | 3.0 |
| Squalan | 6.0 |
| Stearic acid | 2.0 |
| Cholesteryl hydroxystearate | 0.5 |
| Cetyl 2-ethylhexanoate | 4.0 |
| Polyoxyethylene hydrogenated castor oil | 0.5 |
| Self emulsification type glycerin monostearate | 3.0 |
| Potassium hydroxide | 0.15 |
| Sodium hexametaphosphate | 0.05 |

(continued)

| Preparation Example 22: cream | |
|---|---|
| (Formulation) | % by mass |
| Trimethylglycine | 2.0 |
| α-Tocopherol 2-L-ascorbate diesterpotassium phosphate | 1.0 |
| Tocopherol acetate | 0.1 |
| Tencha-extract | 0.1 |
| Paraben | q.s. |
| Trisodium edetate | 0.05 |
| 4-t-Butyl-4'-methoxybenzoylmethane | 0.05 |
| Glyceryl dipara-methoxycinnamate mono-2-ethylhexanoate | 0.05 |
| Colorant | q.s. |
| Carboxy vinyl polymer | 0.05 |
| Purified water | balance |

| Preparation Example 23: cream | |
|---|---|
| (Formulation) | % by mass |
| Liquid paraffin | 8.0 |
| Vaseline | 3.0 |
| Dimethyl polysiloxane | 2.0 |
| Stearyl alcohol | 3.0 |
| Behenyl alcohol | BR>@2.0 |
| Glycerin | 5.0 |
| Dipropylene glycol | 4.0 |
| Dodecyl β-D-glucoside | 3.0 |
| Tetradecyl β-D-glucoside | 3.0 |
| Trehalose | 1.0 |
| Pentaerythrit tetra 2-ethylhexanoate | 4.0 |
| Polyoxyethyleneglyceryl monoisostearate | 2.0 |
| Polyoxyethyleneglycerin monostearate | 1.0 |
| Lipophilic type glycerin monostearate | 2.0 |
| Citric acid | 0.05 |
| Sodium citrate | 0.05 |
| Potassium hydroxide | 0.015 |
| Oil soluble Glycyrrhiza extract | 0.1 |
| Retinol palmitate (a million units) | 0.25 |
| Tocopherol acetate | 0.1 |
| Para-oxybenzoic acid ester | q.s. |
| Phenoxyethanol | q.s. |
| Dibutylhydroxytoluene | q.s. |
| Trisodium edetate | 0.05 |
| 4-t-Butyl-4'-methoxybenzoylmethane | 0.01 |
| 2-Ethylhexyl para-methoxycinnamate | 0.1 |
| β-Carotene | 0.01 |
| Polyvinyl alcohol | 0.5 |
| Hydroxyethylcellulose | 0.5 |
| Carboxy vinyl polymer | 0.05 |
| Purified water | balance |
| Flavor | q.s. |

Preparation Example 24: cream

| (Formulation) | % by mass |
|---|---|
| Liquid paraffin | 8.0 |
| Vaseline | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| Dodecyl β-D-glucoside | 3.0 |
| Trehalose | 1.0 |
| Pentaerythrit tetra 2-ethylhexanoate | 4.0 |
| Polyoxyethyleneglyceryl monoisostearate | 2.0 |
| Polyoxyethyleneglycerin monostearate | 1.0 |
| Lipophilic type glycerin monostearate | 2.0 |
| Citric acid | 0.05 |
| Sodium citrate | 0.05 |
| Potassium hydroxide | 0.015 |
| Oil soluble Glycyrrhiza extract | 0.1 |
| Retinol palmitate (a million units) | 0.25 |
| Tocopherol acetate | 0.1 |
| Para-oxybenzoic acid ester | q.s. |
| Phenoxyethanol | q.s. |
| Dibutylhydroxytoluene | q.s. |
| Trisodium edetate | 0.05 |
| 4-t-Butyl-4'-methoxybenzoylmethane | 0.01 |
| 2-Ethylhexyl para-methoxycinnamate | 0.1 |
| β-Carotene | 0.01 |
| Polyvinyl alcohol | 0.5 |
| Hydroxyethylcellulose | 0.5 |
| Carboxy vinyl polymer | 0.05 |
| Purified water | balance |
| Flavor | q.s. |

Preparation Example 25: cream

| (Formulation) | % by mass |
|---|---|
| Vaseline | 2.0 |
| Dimethylpolysiloxane | 2.0 |
| Ethanol | 5.0 |
| Behenyl alcohol | 0.5 |
| Batyl alcohol | 0.2 |
| Glycerin | 7.0 |
| 1,3-Butylene glycol | 5.0 |
| Dodecyl β-D-glucoside | 1.0 |
| Decyl β-D-glucoside | 1.0 |
| Polyethylene glycol 20000 | 0.5 |
| Jojoba oil | 3.0 |
| Squalan | 2.0 |

(continued)

| Preparation Example 25: cream | |
|---|---|
| (Formulation) | % by mass |
| Phytosteryl hydroxystearate | 0.5 |
| Pentaerythrit tetra 2-ethylhexanoate | 1.0 |
| Polyoxyethylene hydrogenated castor oil | 1.0 |
| Potassium hydroxide | 0.1 |
| Sodium pyrosulfite | 0.01 |
| Sodium hexametaphosphate | 0.05 |
| Stearyl glycyrrhizinate | 0.1 |
| Pantothenyl ethyl ether | 0.1 |
| Albutin | 7.0 |
| Tranexamic acid | 0.1 |
| Tocopherol acetate | 0.1 |
| Sodium hyaluronate | 0.05 |
| Para-oxybenzoic acid ester | q.s. |
| Trisodium edetate | 0.05 |
| 4-t-Butyl-4'-methoxybenzoylmethane | 0.1 |
| Mono 2-ethylhexanoate glyceryl dipara-methoxycinnamate | 0.1 |
| Yellow ferric oxide | q.s. |
| Xantan gum | 0.1 |
| Carboxy vinyl polymer | 0.2 |
| Purified water | balance |

| Preparation Example 26: cream | |
|---|---|
| (Formulation) | % by mass |
| Liquid paraffin | 10.0 |
| Dimethylpolysiloxane | 2.0 |
| Glycerin | 10.0 |
| 1,3-Butylene glycol | 2.0 |
| Undecyl β-D-glucoside | 1.0 |
| Dodecyl β-D-glucoside | 1.0 |
| Erythritol | 1.0 |
| Polyethylene glycol 1500 | 5.0 |
| Squalan | 15.0 |
| Pentaerythrit tetra 2-ethylhexanoate | 5.0 |
| Potassium hydroxide | 0.1 |
| Sodium hexametaphosphate | 0.05 |
| Tocopherol acetate | 0.05 |
| Para-oxybenzoic acid ester | q.s. |
| Hydroxypropylmethylcellulose | 0.3 |
| Polyvinyl.alcohol | 0.1 |
| Carboxy vinyl polymer | 0.2 |
| Acrylic acid/alkyl methacrylate copolymer (Pemulen TR-2) | 0.1 |
| Purified water | balance |

Preparation Example 27: cream

| (Formulation) | % by mass |
| --- | --- |
| Liquid paraffin | 10.0 |
| Dimethylpolysiloxane | 2.0 |
| Glycerin | 10.0 |
| 1,3-Butylene glycol | 2.0 |
| Dodecyl β-D-glucoside | 1.0 |
| Erythritol | 1.0 |
| Polyethylene glycol 1500 | 5.0 |
| Squalan | 15.0 |
| Pentaerythrit tetra 2-ethylhexanoate | 5.0 |
| Potassium hydroxide | 0.1 |
| Sodium hexametaphosphate | 0.05 |
| Tocopherol acetate | 0.05 |
| Para-oxybenzoic acid ester | q.s. |
| Hydroxypropylmethylcellulose | 0.3 |
| Polyvinyl alcohol | 0.1 |
| Carboxy vinyl polymer | 0.2 |
| Acrylic acid/alkyl methacrylate copolymer (Pemulen TR-2) | 0.1 |
| Purified water | balance |

Preparation Example 28: two-layer cream

| (Formulation) | % by mass |
| --- | --- |
| Dimethylpolysiloxane | 5.0 |
| Decamethylcyclopentasiloxane | 25.0 |
| Trimethylsiloxy silicate | 5.0 |
| Polyoxyethylene/methylpolysiloxane copolymer | 2.0 |
| Dipropylene glycol | 5.0 |
| Dodecyl β-D-glucoside | 0.01 |
| Tetradecyl β-D-glucoside | 0.01 |
| Dextrin palmitate-coating particle zinc oxide (60 nm) | 15.0 |
| Dipotassium glycyrrhizinate | 0.02 |
| Glutathione | 1.0 |
| Thiotaurine | 0.05 |
| Clara extract | 1.0 |
| Paraben | q.s. |
| Phenoxyethanol | q.s. |
| Trisodium edetate | q.s. |
| 2-Ethylhexyl para-methoxycinnamate | 7.5 |
| Dimethyldistearylammonium hectright | 0.5 |
| Spherical alkyl polyacrylate powder | 5.0 |
| Butylethylpropane diol | 0.5 |
| Purified water | balance |
| Flavor | q.s. |

Any dermatological external agents of the preparation example 1-28 described above had high wrinkle preventing/ameliorating effect.

Preparation example 29: injection

(Formulation)

| | |
|---|---|
| tetradecyl β-D-glucoside | 100 mg |
| chloroform | 5 ml |
| Tween-80 | 100 mg |
| sterile distilled water | 10 ml |

(Procedure)

100 mg of tetradecyl β-D-glucoside was dissolved in 5 ml of chloroform. 10 mg of Tween-80 was added and mixed then mechanically sterilized with the millipore filter followed by evaporation to dryness with a sterile evaporator. 10 ml of sterile distilled water was added and mixed vigorously, then sonicated and filled into ampuls to prepare an injection. The injection of the preparation example 29 described above had a high ADAM-inhibiting effect.

## Claims

1. An anti-wrinkle agent **characterized by** comprising an alkyl glucoside represented by the general formula below:

[Formula 1]

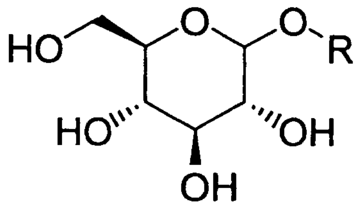

(1)

or a salt thereof wherein R represents a linear or branched alkyl group having 1 to 18 carbon atoms.

2. The anti-wrinkle agent according to claim 1 **characterized in that** R is a linear or branched alkyl group having 10 to 18 carbon atoms.

3. An ADAM inhibitor **characterized by** comprising an alkyl glucoside represented by the general formula (1) below:

[Formula 2]

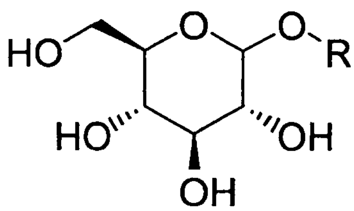

(1)

or a salt thereof wherein R represents a linear or branched alkyl group having 1 to 18 carbon atoms.

4. The ADAM inhibitor according to claim 3 **characterized in that** R is a linear or branched alkyl group having 10 to

18 carbon atoms.

**5.** A method for preventing/ameliorating wrinkles **characterized by** applying the ADAM inhibitor according to claim 3 or 4 to the skin to prevent or ameliorate the wrinkles.

FIG.1

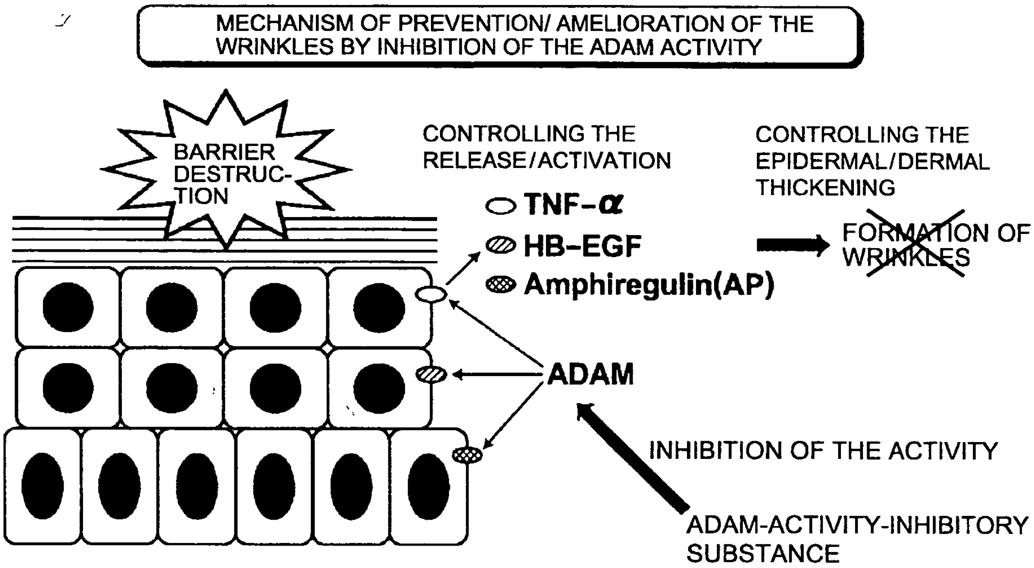
MECHANISM OF PREVENTION/ AMELIORATION OF THE WRINKLES BY INHIBITION OF THE ADAM ACTIVITY
BARRIER DESTRUCTION
CONTROLLING THE RELEASE/ACTIVATION
TNF-α
HB-EGF
Amphiregulin(AP)
CONTROLLING THE EPIDERMAL/DERMAL THICKENING
FORMATION OF WRINKLES
ADAM
INHIBITION OF THE ACTIVITY
ADAM-ACTIVITY-INHIBITORY SUBSTANCE

FIG.2

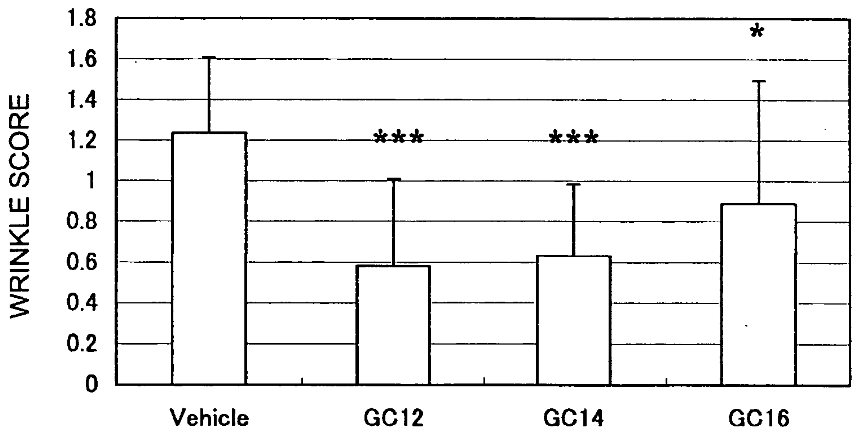
WRINKLE SCORE
1.8
1.6
1.4
1.2
1
0.8
0.6
0.4
0.2
0
***
***
*
Vehicle
GC12
GC14
GC16

FIG.3

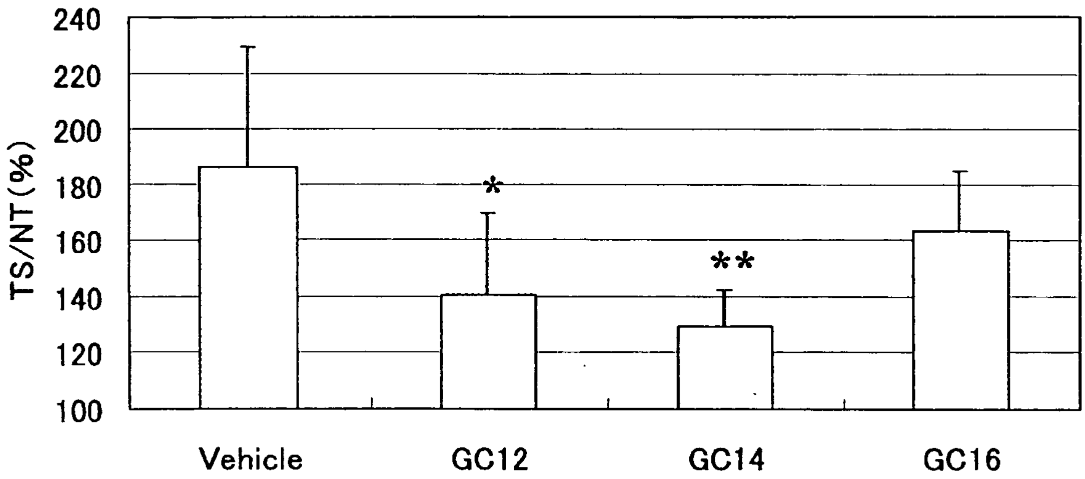
240
220
200
180
160
140
120
100
TS/NT (%)
*
**
Vehicle
GC12
GC14
GC16

FIG.4

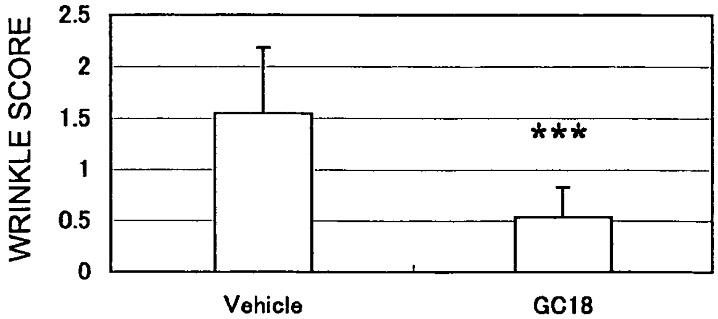
2.5
2
1.5
1
0.5
0
WRINKLE SCORE
***
Vehicle
GC18

FIG.5

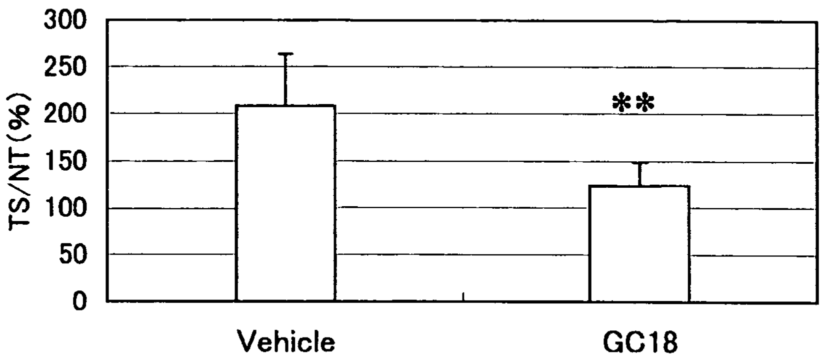
300
250
200
150
100
50
0
TS/NT(%)
**
Vehicle
GC18

FIG.6

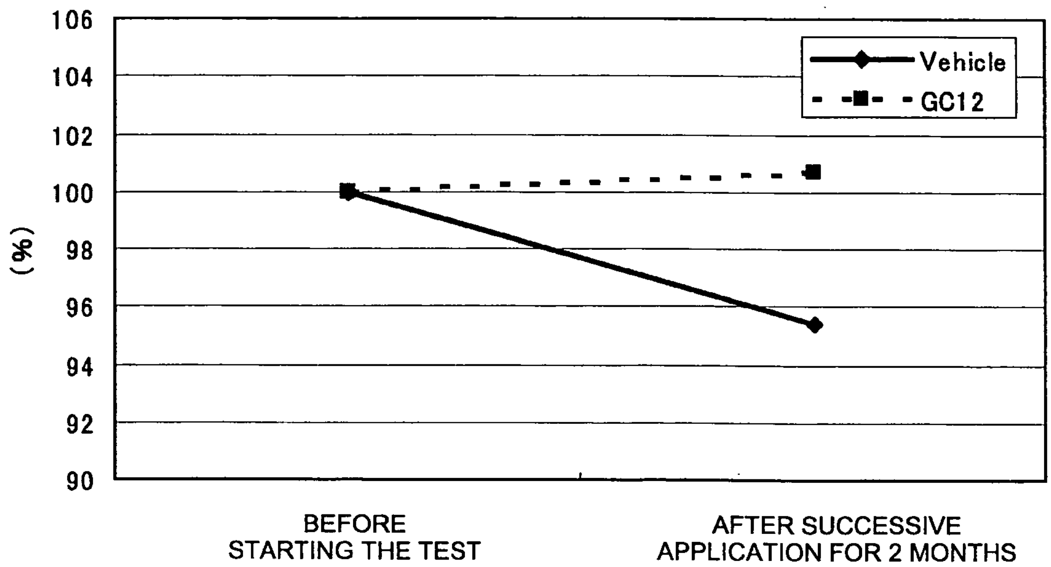
106
104
102
100
98
96
94
92
90
(%)
Vehicle
GC12
BEFORE
STARTING THE TEST
AFTER SUCCESSIVE
APPLICATION FOR 2 MONTHS

**INTERNATIONAL SEARCH REPORT**

International application No.
PCT/JP2007/075120

A. CLASSIFICATION OF SUBJECT MATTER
*A61K8/60*(2006.01)i, *A61K31/7028*(2006.01)i, *A61Q19/00*(2006.01)i, *A61Q19/08*(2006.01)i

According to International Patent Classification (IPC) or to both national classification and IPC

B. FIELDS SEARCHED

Minimum documentation searched (classification system followed by classification symbols)
A61K8/60, A61K31/7028, A61Q19/00, A61Q19/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus/MEDLINE(STN)

C. DOCUMENTS CONSIDERED TO BE RELEVANT

| Category* | Citation of document, with indication, where appropriate, of the relevant passages | Relevant to claim No. |
|---|---|---|
| X | JP 2003-81737 A (KOREANA COSMETICS CO., LTD.), 19 March, 2003 (19.03.03), Par. No. [0001]; example 1 & FR 2829391 A1 & GB 2379386 A & KR 20030021709 A & US 2003/118616 A1 | 1-5 |
| X | JP 2002-80372 A (LOREAL), 19 March, 2002 (19.03.02), Claim 16; example 2 & CA 2354107 A1 & EP 1175888 A2 & FR 2812195 A1 & US 2002/051799 A1 | 1-5 |
| X | JP 2004-168666 A (Kose Corp.), 17 June, 2004 (17.06.04), Par. No. [0007]; examples 1 to 16 (Family: none) | 1-5 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 March, 2008 (07.03.08) | 18 March, 2008 (18.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/075120

C (Continuation). DOCUMENTS CONSIDERED TO BE RELEVANT

| Category* | Citation of document, with indication, where appropriate, of the relevant passages | Relevant to claim No. |
|---|---|---|
| X | JP 61-5005 A (Yoshitomi Pharmaceutical Industries, Ltd.), 10 January, 1986 (10.01.86), Full text (Family: none) | 1-5 |
| X | JP 2-160717 A (Kanebo, Ltd.), 20 June, 1990 (20.06.90), Full text (Family: none) | 1,3,5 |
| P,X | FR 2902334 A1 (Laboratoire Nuxe), 21 December, 2007 (21.12.07), Example 1 (Family: none) | 1-5 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005299524 A
- JP 2005296219 A

### Non-patent literature cited in the description


- **Imokawa et al.** *Fragrance Journal,* 1992, 29-42
- *Hitoshi Masaki, Koryokaisi,* 2001, vol. 25 (1), 34-38
- **Asakura, M. et al.** Cardiac hypertrophy is inhibited by antagonism of ADAM12 processing of HB-EGF: metalloproteinase inhibitors as a new therapy. *Nat. Med.,* 2002, vol. 8, 35-40
- **Izumi, Y. et al.** A metalloprotease-disintegrin, MDC9/meltrin-/ADAM9 and PKC are involved in TPA-induced ectodomain shedding of membrane-anchored heparin-binding EGF-like growth factor. *EMBO J.,* 1998, vol. 17, 7260-7272
- **Lemjabbar, H. ; C. Basbaum.** Platelet-activating factor receptor and ADAM10 mediate responses to Staphylococcus aureus in epithelial cells. *N at. Med.,* 2002, vol. 8, 41-46
- **Sunnarborg, S. W. et al.** Tumor necrosis factor-alpha converting enzyme (TACE) regulates epidermal growth factor receptor ligand availability. *J. Biol. Chem.,* 2002, vol. 277, 12838-12845
- **Yan, Y. ; K. Shirakabe ; Z. Werb.** The metalloprotease Kuzbanian (ADAM10) mediates the transactivation of EGF receptor by G protein-coupled receptors. *J. Cell Biol.,* 2002, vol. 158, 221-226